# EUROPEAN PATENT APPLICATION

(11) **EP 2 444 045 A1**
(43) Date of publication of application: **25.04.2012**
(21) Application number: 10447025.7
(22) Date of filing: 20.10.2010
(51) Int. Cl.: A61F 13/15, B65B 9/04

(54) **Method and apparatus for producing an environmentally friendly absorbent structure**

(71) Applicant: Vynka Bvba, 9220 Hamme (BE)
(72) Inventor: van Ingelgem, Werner, 9220 Hamme (BE)

(57) **Abstract**

The present invention relates to a method and apparatus for forming an composite structure, preferably for use in an absorbent structures used within the personal hygiene industry, such as for instance feminine hygiene garments, baby diapers and pants and adult incontinence garments. The present invention preferably provides a method and apparatus for depositing particulate material in a desired pattern onto a moving carrier layer. The method allows accurate forming of a pre-determined pattern of particulate material clusters at high production speed, with reduced raw material usage and relative low cost. As such method allows manufacturing of absorbent structures being substantially cellulose free and substantially glue free, considered technically, economically and environmentally friendly. The present invention foresees in the need for an improved thin, flexible, lightweight particulate material absorbent structure which overcomes the problems of the prior art having optimal absorption, distribution and retention.

## Description

### FIELD OF THE INVENTION

The present invention relates to a method and apparatus for manufacturing a sandwich structure consisting of a carrier layer, an auxiliary layer and absorbent particulate polymer material positioned there between. The method allows depositing absorbent particulate polymer material positioned in a desired pre-determined pattern of absorbent particulate polymer material positioned clusters onto a moving carrier layer, adding an auxiliary layer and subsequently attaching the layers to each other to form a sandwich structure at high production speed and relative low cost. Such method is particularly useful in the manufacture of absorbent composites for use in absorbent structure of absorbent article, preferably a disposable article, such as a feminine hygiene garment, baby diaper, baby pants and adult incontinence garment. As such method allows manufacturing absorbent structures without substantial use of cellulose fibers and/or chemical binders, it is considered to be very environmentally friendly.

### BACKGROUND OF THE INVENTION

There has been increasing demand in recent years for flexible, thinner, lightweight absorbent structures to resolve various problems of manufacturing, marketing, design, fit, wearing comfort, distribution, garbage disposal, material and energy consumption, transportation and storage costs and the like.

It is known from the art to introduce absorbent particulate polymer material into a matrix of cellulose fibers so as to create an absorbent structure whereby the absorbent particulate polymer material is capable of absorbing, distributing and retaining liquid while the cellulose fiber matrix acts as a grid, keeping absorbent particulate polymer material in its desired location. However, since the presence of cellulose fibers, usually wood pulp or fluff, results in relatively thick and bulky products, the process of manufacturing fluff involves cutting down trees and treating the wood pulp with various chemicals and transportation of the raw materials and end product is typically done over relative large distances, the product and process is considered to be less environmentally friendly. Hence there has been a desire to manufacture such absorbent structures without the use of wood pulp.

Many attempts have been undertaken to manufacture flexible, thin and lightweight absorbent structures, consisting of an absorbent material bonded to or in between one or more carrier layers. The physical and/or chemical interaction in between the absorbent material, the bonding material and/or the carrier layer often leads to a reduced absorption, distribution and/or retention performance despite functional and/or structural requirements.

It has been found that it is important to at least partially immobilise the absorbent particulate polymer material by compartmentalizing, restraining and/or bonding it to the absorbent structure in such a way that the absorbent structure is able to hold the absorbent particulate polymer material in either dry, partially and/or fully liquid loaded state. Failing to provide sufficient structural integrity results in loss of functional performance such as absorbent structure coherence, absorption, distribution, transport and/or retention performance and partial or overall failure.

In order to be able to keep absorbent particulate polymer material compartmentalized, restrained and/or bonded within the absorbent structures during usage, it is necessary to deposit such absorbent particulate polymer material in a predetermined and desired location, grid or pattern during the manufacturing process. It is therefore very desirable to enable the depositing of absorbent particulate polymer material in a substantially continuously, specific, well-defined and discretely arranged pattern onto a carrier layer moving at a relatively high speed.

Multiple attempts have been made to provide methods to manufacture composite structures with selected regions of particulate materials intermittently and discretely located along the length of a carrier layer, whereby EP1621166 describes a process for forming a sandwich structure having a pattern of particulate material enveloped between a carrier and cover material. The method comprises the steps of providing an essentially flat carrier material and an essentially flat cover material for forming the outer sandwich layers. Further steps provide an essentially endless support means for the carrier material having a support pattern and a carrier material holding means. The carrier material is positioned over the support means at a carrier speed relative to a fixed frame, whereby the carrier means contacts the support pattern with its support surface and whereby the relative speed between carrier material and the contact surface of the carrier support means is essentially zero. An absorbent particulate material is provided and an amount of absorbent particulate material is pre-metered. The cover material and the carrier material are combined and affixed by a fixation means with the absorbent particulate material positioned there between. The carrier material is supported only in the region of the support pattern of the support means and deformed by a carrier holding means such that indentations are formed in the unsupported regions. The absorbent particulate material is fanned into the indentations of the carrier material; thereby forming a primary pattern of particulate material which is sandwiched in between the outer layers.

Whilst the prior art attempts describe approaches to manufacture sandwich structures and mechanism to obtain discrete particulate material pockets, it is believed that the above methods suffer from one or more drawbacks. The prior art methods lack accuracy and repeatability of the absorbent particulate material patterns and have unbalanced pre-metering, forming and transport means. The large and expensive pre-metering, forming and suction means required for the particulate material transport and clustering require very specific customization for each product concept/size/absorbency and are very expensive to design, build, install and replace. These apparatus are furthermore consuming a relative high amount of energy and take significant resources to operate during normal production. The web materials require operating within very narrow tolerances in relation to particle material clustering and depositing and can only operate under relatively limited production speeds, demanding large and complex machinery and installations which are subject to excessive tear, wear, maintenance, cleaning, adjustment, etc. The immobilisation and/or fixation means in prior art products involves using chemical adhesives, glues or binders, which have a negative impact on the environment and add extra cost to the process. Also, the prior art methods require the carrier layer to be at least sufficiently gas permeable, whilst it would be desired for many applications in fluid handling and storing products such as e.g. baby diapers that the carrier layer would be composed of a liquid and substantially gas impervious barrier layer. Such inefficient and ineffective methods and the complex manufacturing processes makes neither of the prior art methods economically, technically and/or environmentally advantageous.

Hence, there is still a need in the art for a reliable, environmentally-friendly, cost-efficient and maintenance-friendly method and apparatus to consistently dispose and pocket particulate materials continuously in a specific, well-defined and discretely arranged pattern on the surface of a carrier layer which moves at a high production speed with limited consumption and/or wastage of resources during the production process.

As a result of exhaustive research to address the above-identified and related problems, the inventors have found a more favorable and advantageous manufacturing process to obtain such concepts and structures, which will be explained in greater detail down below.

### SUMMARY OF THE INVENTION

The present invention relates to a method for manufacturing a composite structure having a particulate material, preferably absorbent particulate polymer material, deposited in a predefined and desired pattern onto a carrier layer so as to form substantially cellulose free and substantially glue free absorbent structures!

In particular, the present invention provides a method for producing a sandwich structure comprising a pattern of particulate material, said method comprising the steps of:
a - providing at least one essentially endless layer as carrier layer and/or auxiliary layer
b - providing an essentially endless support means for said carrier layer,
c - positioning said carrier layer over said support means,
   whereby said carrier layer is in contact with the contact surface of said support means, and whereby the relative speed between said carrier layer, contact surface of
   said carrier support means and the clustering means is essentially zero;
d - providing a particulate material stream from a particle material supply means;
e - directing the particulate material through the perforations of a clustering means;
f - providing an essentially endless material as auxiliary material;
g - combining said auxiliary layer and said carrier layer with said particulate material sandwiched there between;
   characterized in that,
g - said clustering means deposits said particulate material in a pre-designated locations onto the carrier layer thereby creating a printing pattern of particulate material onto said carrier layer, thereby forming a primary pattern of particulate material and
i - providing an ultrasonic or heat sealing sandwich immobilisation means,

The method comprises the steps of providing an essentially endless carrier layer in a particulate material deposit position in close proximity to a clustering means. In a preferred embodiment of the invention the clustering means with desired perforation patterns are incorporated into an essentially endless rotating drum, the clustering means can be mounted replaceably onto the rotating drum or can be unitary therewith. The carrier layer is positioned over a substantially endless support means at a carrier speed relative to a fixed frame, whereby the carrier layer is in contact with the support means and whereby the relative speed between carrier layer, the support means and the clustering means and/or drum is preferably essentially zero. A particulate material is provided by a particulate material supply means and the particulate material stream is directed towards the clustering means. The particulate material is collected by the inlet regions of the perforations of the clustering means and held in the outlet region and/or inlet regions of the perforations of the clustering means until evacuated. The carrier layer is preferably supported over at least part of the major support means surface area while the particulate material caught and held in the perforations is deposited onto the carrier material.

In an embodiment according to the invention, the resulting composite structure of a carrier layer with discretely deposited particulate material clusters thereon, is complemented with an auxiliary layer, such as for instance non-woven, tissue, paper, thermoplastic material and the like and/or affixed by attachment means, such as for instance glue, bonds, joints and the like, with the particulate material clusters relatively immobilized there between so as to obtain a sandwich structure usable in the form of an absorbent structure.

In a preferred embodiment the auxiliary layer immobilizes and/or restrains the particulate material clusters and/or the attachment means seal, bond and/or join the outer layers together via ultrasonic bonding, thermo-bonding, and/or pressure-bonding means. The attachment means preferably form and/or define pockets or compartments of particulate material clusters, whereby the bonding and/or joint regions comprise essentially no particulate material and/or glue. For economical, environmental and technical reasons the particulate material clusters are preferably not intentionally covered with substantial amounts of thermoplastic materials, glues, binders and/or adhesives to fixate, pocket, encapsulate, bind and/or join these particulate material clusters to one or more outer layers, but the particulate material clusters are preferably pocketed by providing an attachment pattern in between the outer-layers by way of attachment means such as ultra sonic bonding means.

Additional materials and/or layers may be provided to provide extra functional and/or structural advantages such as strength, acquisition, absorption, distribution, transport, retention, etc. may be incorporated.

Another aspect of the present invention provides an apparatus for producing a composite structure comprising particulate material clusters, said apparatus comprising:
a - a clustering means having a desired perforation pattern
b - a particulate material supplying means positioned to provide particulate materials into the inlet regions of the perforations of said clustering means;
c - a carrier layer supplying means for providing a carrier layer; support means for moving said carrier layer in close proximity to the outlet regions of the clustering means;
d - transport means for moving said carrier layer with particulate material clusters away from the clustering means.
e - covering means for providing an auxiliary layer over said particulate material clusters.

The invention provides an apparatus for forming a composite structure having predetermined and selected particulate material clusters deposited on a carrier layer. The apparatus comprises a clustering means having perforations comprising inlet regions and outlet regions, and a particulate material supplying means providing particulate materials to the clustering means. The carrier layer supplying means provides a carrier layer, while the support means move the carrier layer into depositing position of the outlet regions of the clustering means. The clustering means are preferably arranged to catch, collect, hold and deposit the particulate materials from the particulate material stream as provided by the particulate supply means. The perforations within the clustering means are designed so as to catch and collect the particulate material stream by way of the inlet regions, and preferably converge the particulate materials towards the outlet regions so as to accumulate and build up particulate material clusters within the perforations ready for deposit onto the carrier layer in preferably discrete printing patterns of particulate material clusters. The support means brings the carrier layer into a depositing position of, preferably in close proximity to, most preferably in contact with, the outlet regions of perforations of the clustering means. Preferably a selected level of pressure contact in between the deposit surface of the carrier layer and the outer contact surface of the clustering means is provided, so as to lock the discrete particulate material clusters until deposited onto the deposit surface of the carrier layer. In an alternative embodiment, no substantial pressure contact in between the deposit surface of the carrier layer and the outer contact surface of the clustering means is provided, and the particulate material provided via the particulate material stream is immediately caught, collected and deposited upon the carrier layer. A transport means moves the carrier layer and the particulate materials deposited thereon away from the clustering means. In a preferred embodiment the particulate material clusters deposited onto the carrier layer are covered by an auxiliary layer via a covering means so as to form for instance an absorbent polymer material area. In a more preferred embodiment, the particulate material clusters from the composite structure are relatively immobilised, bound, joined and/or otherwise restrained in between a carrier layer and any suitable auxiliary layer. Preferably, attachment means leading to a composite sandwich structure of a carrier layer and auxiliary layer with particulate materials enclosed and/or immobilised, bound, join and/or restrained there between in the form of compartment or clusters are provided.

In a preferred embodiment, the particulate material supply means comprises a vibrating, coiling and/or turning means so as to accurately, continuously and controllably provide the required particulate material amounts, sizes and/or speeds towards the clustering means. It is furthermore preferred, that the particulate material is being transported in between such vibrating, coiling and/or turning means and the clustering means by way of a feeding tube means, which can alternatively be arranged with gas pressure means to guide and direct the particulate material stream to the inlet regions of the clustering means. In a preferred embodiment, the feeding tube is substantially longitudinal, vertically and/or converging and the particulate material has a substantial weight so that one can use gravity to transport the particles through the feeding tube into the clustering means. However, additional conveyer means 225 such as mechanical (such as airflows) or electro-magnetic (such as magnets in case the particles interact with a magnetic field) and/or other means can be used to convey and/or help convey such particulate material stream towards the clustering means.

In a preferred embodiment, a dosing means of a volumetric, gravimetric or other type so as to control the quantity, size and/or speed of particles entering the feeding tube means is provided. It is further preferred that the particulate material stream can be redirected via the control means into removing means so as to be moved again towards the particulate supply means via recovery tubing means for re-use and/or less preferably guided out of the production system via collection means to be moved towards separate storage facilities for later usage and/or disposal in case irreparably damaged, contaminated, spoiled and/or rendered unusable.

In a preferred embodiment, the clustering means are unitary with a substantially endless rotating drum, preferably having a cylinder-like outer shell. In a preferred embodiment an endless rotating drum with replaceable, customisable and/or adjustable clustering means is provided. The drum is running in line with the opening of the feeding tube means thereby directing the particulate materials to fall, be guided, directed, pushed and/or vacuumed through the respective inlet regions and/or outlet regions of the perforation pattern of the clustering means to obtain the desired printing pattern.

It is preferred to under-fill (or, eventually 'right-fill') the perforations of the clustering means. Using a particle material supply means with a preferably gravimetric dosing system in combination with a feeding tube means such as a tube, pipe or conveyer of the right dimensions and shape will then ensure that the proper weights are supplied, gathered in the perforations and deposited by the outlet regions so as to form the desired printing pattern of particulate material clusters as stipulated by the perforation pattern of the clustering means.

In a preferred embodiment, the inlet regions are of a funnel-like shape with steep slopes separated from one another by sharp ridges so no substantial amounts of particulate materials remain caught in between the inlet regions and/or on the slopes of the inlet regions, but are readily transported towards the outlet regions for scheduled deposit on the carrier material. In a preferred embodiment, a combination of larger and smaller inlet regions and outer regions are provided leading to homogenous, heterogeneous or complex perforation patterns.

Preferably the void volume of the perforations is larger than the volumes of particulate material one wants to print, to prevent overfilling of the clustering means during the production process. In this case of correctly designed clustering means and a fully, effectively and efficiently working apparatus one can also greatly increase accuracy and reliability of the manufacturing process and thereby also eliminating the need for a sweeping means, thus optimising raw material usage, limiting investment cost and reducing maintenance cost. However in case required, any 'overfill', migration and/or misplacement of particulate material can be guided and/or redirected via sweeping means, such as scrapers, brushes, air blow, vacuum suction, etc, into one or more available inlet regions of the cluster means and/or be guided into recovery means or collection means so as to be moved away from the clustering means for re-use, collection and/or disposal.

The method and apparatus according to a preferred embodiment of the invention leads to highly appreciated thin, flexible and/or light-weight composite structure and absorbent structures which are economically, environmentally, technically and/or commercially advantageous, not in the least since they are obtained without substantial and bulky amounts of fibrous absorbent materials such as fluff and wood pulp (cfr "fluffless") and are not using substantial and expensive amounts of glue, binder, adhesive and/or other thermoplastic materials (cfr "glueless"). This has so far been unprecedented within the prior art.

### BRIEF DESCRIPTION OF FIGURES

**Figure 1** provides a schematic process diagram for carrying out the invention.
**Figure 2** provides a schematic top view illustration of a clustering means mounted onto a drum according to an embodiment of the invention.
**Figure 3** provides a schematic top view illustration of a clustering means according to an embodiment of the invention.
**Figure 4** provides schematic top view illustration of a carrier layer with deposited particulate material clusters according to a printing pattern according to an embodiment of the invention.
**Figure 5** provides a cross-sectional schematic illustration of a clustering means according to an embodiment of the present invention.
**Figure 6** provides a cross-sectional schematic illustration of a printed particulate material cluster according to an embodiment of the present invention.
**Figure 7** provides schematic illustration of an apparatus for carrying out the invention according to an embodiment of the invention.
**Figure 8** provides schematic enlarged sectional view of a part of the equipment for the process as shown in Figure 7.
**Figure 9** is a top plan view of a diaper as a preferred embodiment of an absorbent article according to the invention, with the upper layers partially cut away.

### DETAILED DESCRIPTION OF THE INVENTION

The present invention relates to a method and apparatus for creating composite structures comprising particulate material, preferably absorbent particulate material such as absorbent polymer materials, more preferably absorbent particulate polymer material; preferably clustered, enveloped and/or immobilised in between a carrier layer and auxiliary layer, possibly via attachment means, so as to form discrete and predetermined printing patterns of particulate material sheets for use in absorbent products, preferably a disposable absorbent article from the personal hygiene industry, such as feminine hygiene garments, baby diapers and pants and adult incontinence garments.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. However, it is to be understood that the value to which the modifier "about" refers is itself also specifically disclosed.
"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.
"Absorbent component" as used herein refers to a structural constituent of an absorbent structure, e.g., a piece of an absorbent core, such as one of multiple pieces in a multi-piece absorbent core.
"Absorbent element" as used herein refers to a part of a functional constituent of an absorbent structure, e.g., a liquid acquisition layer, a liquid distribution layer, or a liquid storage layer formed of a material or materials having particular liquid handling characteristics suitable for the specific function.
"Absorbent insert" as used herein refers to a device adapted for insertion into an absorbent article and to serve as an absorbent structure when so inserted.
"Absorbent layer" as used herein refers to a term referring to a discrete, identifiable sheet-like or web-like element of an absorbent structure which may remain detached and relatively movable with respect to another such element or may be attached or joined so as to remain permanently associated with another such element. Each absorbent layer may itself include a laminate or combination of several layers, sheets and/or webs of similar or diverse compositions.
"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01 ).
"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g. bond area's) or unintentional (e.g. manufacturing artefacts).
"Absorbent particulate polymer material" as used herein refers to an absorbent polymer material which is in particulate form such as powders, granules, flakes and the like so as to be flowable in the dry state.
"Absorbent structure" as used herein refers to those elements of an absorbent article comprising material or a combination of materials suitable to absorb, distribute and retain bodily exudates.
"Absorption" as used herein refers to the process by which a liquid is taken up within a material.
"Acquisition layer", "acquisition region", "acquisition surface" or "acquisition material" and the like as used herein refer to a layer having a faster liquid uptake capability.
"Absorbency" is the ability of a material to take up fluids by various means including capillary, osmotic, solvent, chemical or other action.
"Adult incontinence garment" as used herein refers to absorbent articles intended to be worn by incontinent adults, for absorbing and containing bodily exudates.
"Adhesion" as used herein refers to the force that holds different materials together at their interface.
"Adhesive" as used herein refers to a material, which may or may not be flowable in solution or when heated, that is used to bond materials together.
"Adsorption" as used herein refers to the process by which a liquid is taken up by the surface of a material.
"Air laying" as used herein refers to forming a web by dispersing fibres or particles in an air stream and condensing them from the air stream onto a moving screen by means of a pressure or vacuum; a web of fibres produced by air laying is herein referred to an "airlaid"; an airlaid web bonded by one or more techniques to provide fabric integrity is herein referred to an "airlaid non-woven".
"Apparent density", "density" and like as used herein refers to the basis weight of the sample divided by the calliper with appropriate unit conversions incorporated therein. Apparent density used herein has the unit g/cm³.
"Attach", "attached" and "attachment" as used herein are synonymous with their counterparts of the terms "fasten", "affix", "secure", "glue", "bind", "join" and "link".
"Baby diaper" as used herein refers to absorbent articles intended to be worn by children, for absorbing and containing bodily exudates which the user draws up between the legs and fastens about the waist of the wearer.
"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.
"Back region" as used herein refers to the portion of an absorbent article or part thereof that is intended to be positioned proximate the back of a wearer.
"Backing" as used herein refers to a web or other material that supports and reinforces the back of a product.
"Basis weight" is the weight per unit area of a sample reported in grams per square meter, g/m² or gsm.
"Bodily exudates", "body exudates", "bodily fluids", "body fluids", "bodily discharges", "body discharges", "liquids" and the like as used herein are used interchangeably and refer to, but are not limited to urine, blood, vaginal discharges, breast milk, sweats and faecal matter.
"Binder", "adhesive", "glue", "resins", "plastics" and the like as used herein are used interchangeably and refer to substances, generally in a solid form (e.g. powder, film, fibre) or as a foam, or in a liquid form (e.g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.
"Bond strength" as used herein refers to the amount of adhesion between bonded surfaces. It is a measure of the stress required to separate a layer of material from the base to which it is bonded.
"Capillary action", "capillarity", or "capillary motion" and the like as used herein are used to refer to the phenomena of the flow of liquid through porous media.
"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.
"Cellulose fibres" as used herein refers to naturally occurring fibres based on cellulose, such as, for example cotton, linen, etc; wood pulp fibres are one example of cellulose fibres; man-made fibres derived from cellulose, such as regenerated cellulose (rayon), or partially or fully acetylated cellulose derivatives (e.g. cellulose acetate or triacetate) are also considered as cellulose fibres.
"Cluster" or the like as used herein refers to an agglomeration of particles and/or fibres.
"Chemically stiffened fibres", chemically modified fibres", "chemically cross-linked fibres", "curly fibres" and the like as used herein are used interchangeably and refer to any fibres which have been stiffened by chemical means to increase stiffness of the fibres under both dry and aqueous conditions, for example by way of addition of chemical stiffening agents (e.g. by coating, impregnating, etc), altering the chemical structure of the fibres themselves (e.g. by cross-linking polymer chains, etc) and the like.
"Cohesion" as used herein refers to the resistance of similar materials to be separated from each other.
"Compartment" as used herein refers to chambers, cavities, pockets and the like.
"Comprise," "comprising," and "comprises" and "comprised of' as used herein are synonymous with "include", "including", "includes" or "contain", "containing"., "contains" and are inclusive or open-ended terms that specify the presence of what follows e.g. a component and do not exclude or preclude the presence of additional, non-recited components, features, elements, members, steps, known in the art or disclosed therein.
"Coverstock" as used herein refers to a lightweight non-woven material used to contain and conceal an underlying absorbent core material; examples are the facing layer or materials that cover the absorbent cores of feminine hygiene garments, baby diapers and pants and adult incontinence garments.
"Crotch region" of an absorbent article as used herein refers to about 50% of the absorbent article's total length (i.e., in the y-dimension), where the crotch point is located in the longitudinal centre of the crotch region. That is, the crotch region is determined by first locating the crotch point of the absorbent article, and then measuring forward and backward a distance of 25% of the absorbent article's total length.
"Cross direction (CD)", "lateral" or "transverse" and the like as used herein are used interchangeably and refer to a direction which is orthogonal to the longitudinal direction and includes directions within ±45° of the transversal direction.
"Curing" as used herein refers to a process by which resins, binders or plastics are set into or onto fabrics, usually by heating, to cause them to stay in place; the setting may occur by removing solvent or by cross-linking so as to make them insoluble.
"Diaper", "conventional diaper", "diaper-like", "diaper-like garment" and the like as used herein are used interchangeably and refer to disposable absorbent articles, which typically include a front waist portion and a back waist portion which may be releasably connected about the hips of the wearer during use by conventional fasteners such as adhesive tape fasteners or hook and loop type fasteners. In use, the article is positioned between the legs of the wearer and the fasteners are releasably attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Distribution layer", "distribution region", "distribution surface" or "distribution material" and the like as used herein are used interchangeably and refer to a layer having a larger capacity in wicking, dispersing and distributing liquids.
"Dry laying" as used herein refers to a process for making a non-woven web from dry fibre; these terms apply to the formation of carded webs, as well as to the air laying formation of random webs; a web of fibres produced by dry laying is herein referred to as a "drylaid"; a drylaid web bonded by one or more techniques to provide fabric integrity is herein referred to a "drylaid non-woven".
"Dry strength" as used herein refers to the strength of an adhesive joint determined in dry state conditions, immediately after drying under specified conditions or after a period of conditioning in the standard laboratory atmosphere.
"Fabric" as used herein refers to a sheet structure made from fibres, filaments and/or yarns.
"Feminine hygiene garments" as used herein refer to absorbent hygiene articles intended to be worn by woman, for absorbing and containing body exudates.
"Fibre" as used herein refers to the basic threadlike structure from which non-woven, yarns and textiles are made. It differs from a particle by having a length at least 4 times its width; "Natural fibres" are either of animal (wool, silk), vegetable (cotton, flax, jute) or mineral (asbestos) origin, while "Man-made fibres" may be either polymers synthesised from chemical compounds (polyester, polypropylene, nylon, acrylic etc.) or modified natural polymers (rayon, acetate) or mineral (glass). "Fibre" and "filament" are used interchangeably.
"Film", "foil" and the like as used herein are used interchangeably and refer to a thin sheet of essentially non-absorbent material such as plastic or closed foams. In this invention it particularly refers to materials that do not correspond to non-woven.
"Fluff pulp" as used herein refers to wood pulp specially prepared to be dry laid.
"Front region" as used herein refers to the portion of an absorbent article or part thereof that is intended to be positioned proximate the front of a wearer.
"Garment facing layer" as used herein refers to elements of the chassis that form the outer surface of the absorbent article, such as the back sheet, the side panels, the waist fasteners, and the like, when such elements are present.
"Heat activated adhesive" as used herein refers to a dry adhesive that is rendered tacky or fluid by application of heat or heat and pressure to the assembly.
"Heat sealing adhesive" as used herein refers to a thermoplastic adhesive which is melted between the adherent surfaces by heat application to one or both of the adjacent adherent surfaces.
"Highloft" as used herein refers to general term of low density, thick or bulky fabrics.
"Hot-melt adhesive" as used herein refers to a solid material that melts quickly upon heating, then sets to a firm bond upon cooling; used for almost instantaneous bonding.
"Hydrophilic" as used herein refers to having an affinity for being wetted by water or for absorbing water.
"Hydrophobic" as used herein refers to lacking the affinity for being wetted by water or for absorbing water.
"Immobilisation layer" as used herein refers to a layer able to be applied to the particulate material with the intent to immobilize, bond, join and/or restrain particulate material.
"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.
"Knitting" as used herein refers to the technique for interlocking loops of fibres with needles or similar devices.
"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and faces the wearer facing layer; conversely, the term "lower" refers to the layer of the absorbent article which is nearest to and faces the garment facing layer. "Layer" is three dimensional structure with a x dimension width, y dimension length, and z-dimensions thickness or calliper, said x-y dimensions being substantially in the plane of the article, however it should be noted that the various members, layers, and structures of absorbent articles according to the present invention may or may not be generally planar in nature, and may be shaped or profiled in any desired configuration.
"Machine direction (MD)", "longitudinal" and the like as used herein are used interchangeably and refer to a direction running parallel to the maximum linear dimension of the structure and includes directions within ±45° of the longitudinal direction.
"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.
"Mass flow" as used herein refers to the flow of a liquid from one absorbent element or component to another absorbent element or component by channel flow action.
"Mechanical bonding" as used herein refers to a method of bonding fibres by entangling them. This can be achieved by needling, stitching with fibres or by the use of high-pressure air or water jets and the like.
"Non-woven" as used herein refers to manufactured sheet, web or batt of directionally or randomly orientated fibres, bonded by friction, and/or cohesion and/or adhesion, excluding paper and products which are woven, knitted, tufted, stitch-bonded incorporating binding yarns or filaments, or felted by wet-milling, whether or not additionally needled. The fibres may be of natural or man-made origin and may be staple or continuous filaments or be formed in situ. Commercially available fibres have diameters ranging from less than about 0.001 mm to more than about 0.2 mm and they come in several different forms: short fibres (known as staple, or chopped), continuous single fibres (filaments or monofilaments), untwisted bundles of continuous filaments (tow), and twisted bundles of continuous filaments (yarn). Non-woven fabrics can be formed by many processes such as melt blowing, spun bonding, solvent spinning, electro spinning, and carding. The basis weight of non-woven fabrics is usually expressed in grams per square meter (gsm).
"Pant", "training pant", "closed diapers", "prefastened diapers", "pull-on diapers" and "diaperpants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).
"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.
"Rear" as used herein refers to the portion of an absorbent article or part thereof that is intended to be positioned proximate the back of the wearer.
"Resin" as used herein refers to a solid or semisolid polymeric material.
"Substantially cellulose free" as used herein refers to an absorbent article, structure or core, that contains less than 20% by weight cellulosic fibres, less than 10% cellulosic fibres, less than 5% cellulosic fibres, no cellulosic fibres, or no more than an immaterial amount of cellulosic fibres which do not materially affect the thinness, flexibility or absorbency thereof.
"Thermobonding" as used herein refers to a method of bonding fibres by the use of heat and/or high-pressure.
"Thermoplastic" as used herein refers to polymeric materials that have a melting temperature and can flow or be formed into desired shapes on the application of heat at or below the melting point.
"Ultrasonic" as used herein refers to the use of high frequency sound to generate localised heat through vibration thereby causing thermoplastic fibres to bond to one another.
"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.
"Wearer facing layer" as used herein refers to elements of the chassis that form the inner surface of the absorbent article, such as the topsheet, the leg cuffs, and the side panels, etc., when such elements are present.
"Weaving" as used herein refers to the process of interlacing two or more sets of yarns at right angles to form a fabric; a web of fibres produced by weaving is herein referred to as a "Woven".
"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web. Without intending any limitation, such web materials may be cellulosic fibre materials, tissues, woven or non-woven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, non woven / film laminates. Web materials may comprise other materials, such as added binding material, particles, hydrophilizing agents and the like.
"Wet burst strength" is a measure of a layer's ability to absorb energy, when wet and subjected to deformation normal to the plane of the web.
'Wet strength" as used herein refers to the strength of an adhesive joint determined immediately after removal from a liquid in which it has been immersed under specified conditions of time, temperature and pressure. The term is commonly used in the art to designate strength after immersion in water.
"Wet laying" as used herein refers to the forming a web from an aqueous dispersion of fibres by applying modified paper making techniques; a web of fibres produced by wetlaying is herein referred to as a "wetlaid".
"Wood pulp" as used herein refers to cellulosic fibres used to make viscose rayon, paper and the absorbent cores of products such as feminine hygiene garments, baby diapers and pants and adult incontinence garments.
"X-y dimension" as used herein refers to the plane orthogonal to the thickness of the article, structure or element. The x- and y-dimensions correspond generally to the width and length, respectively, of the article, structure or element.
"Z-dimension" as used herein refers to the dimension orthogonal to the length and width of the article, structure or element. The z-dimension corresponds generally to the thickness of the article, structure or element.

The recitation of numerical ranges by endpoints includes all numbers and fractions subsumed within that range, as well as the recited endpoints. Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Upon review of the Detailed Description of the Invention and the accompanying Drawings provided herein, it will be apparent to one of ordinary skill in the art that the method and apparatus according to the present invention may be used to obtain composite structures, absorbent structures which may be used in absorbent articles, and more particularly, in disposable absorbent articles, such as feminine hygiene garments, baby diapers and pants and adult incontinence garments. Accordingly, the present invention shall not be limited to the method, apparatus, absorbent structures and absorbent articles specifically described and illustrated herein, although the following description is particularly directed to manufacture of composite structures and absorbent structure for absorbent disposable baby diaper products.

The present invention was based upon the findings that products resulting from a method and apparatus to create a composite structure comprising particulate material deposited in a predesigned and desired pattern, can be more efficiently used to absorb, distribute and retain liquid. Absorbent articles, such as feminine hygiene garments, baby diapers and pants and adult incontinence garments absorb, distribute, transport and contain bodily exudates. They also are intended to prevent bodily exudates from leaking, soiling, wetting or otherwise contaminating clothing or other articles, such as bedding, that come in contact with the wearer. A disposable absorbent article, such as a disposable diaper, may be worn for several hours in a dry state or in bodily exudates loaded state. Accordingly, efforts have been made toward improving the fit and comfort of the absorbent article to the wearer, both when the article is dry and when the article is fully or partially loaded with body exudates, while maintaining or enhancing the absorbing and containing functions of the article.

Some absorbent articles, like diapers, contain a particulate material, such as an absorbent polymer material, more preferably an absorbent particulate polymer material. Although such absorbent particulate material absorbs liquid and swells, it is more effective when disposed in an absorbent structure in a certain pattern or arrangement when intending optimal absorbency, fit and/or comfort. Since it is desirable for absorbent particulate material to remain in its intended location in an absorbent article, such particulate materials are to be clustered and are desirably relatively immobilized in the absorbent article such that the absorbent particulate material remains immobilized, bonded, joined and/or restrained on their intended location when the absorbent article is dry, partially wetted and/or fully wetted.

In addition to being absorbent, absorbent articles, such as diapers, may desirably also be thin, flexible and/or light-weight, for ease and comfort in use and for more convenient and neat packaging and storage. Absorbent articles, which may often be used in large quantities, may also desirably be inexpensive. Some technologies of clustering and immobilizing absorbent particulate polymer material in an absorbent article such as additional fluff, add bulk to the absorbent article and thereby increase thickness, reduce flexibility, and/or increase cost of the absorbent article. Other technologies for immobilizing absorbent particulate polymer material in an absorbent article may not be as effective in maintaining immobilization when the absorbent article is in the wet state as when in the dry state. Accordingly, there remains a need for a method and apparatus which can dose the absorbent particulate material in specified quantities, cluster it in desired particulate material clusters and deposit it in a desired pattern so that thin, flexible, and/or inexpensive absorbent article can be obtained.

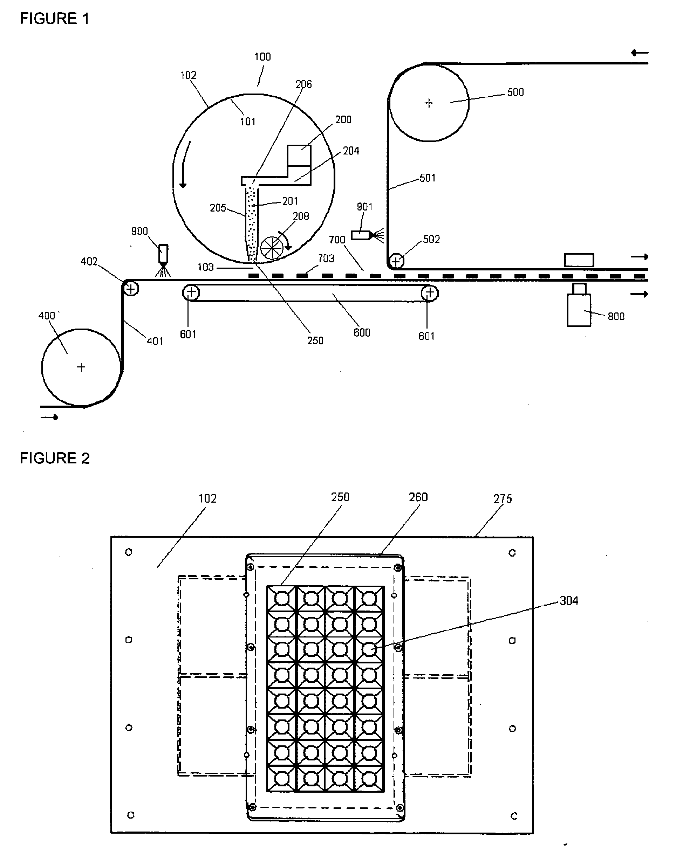

With reference to Figure 1, the present invention can provide an apparatus for forming a composite structure 700 having a plurality of discrete particulate material clusters which are preferably discretely distributed and deposited on a carrier layer 401 and contain selected quantities of particulate material 201. The representatively shown apparatus includes a clustering means 250 with perforations 304, and a particulate material supplying means 200 for providing particulate materials 201 towards the clustering means 250. A web supplying means 400 provides a carrier layer 401 and a support means 600 moves the carrier layer 401 adjacent the clustering means 250, the clustering means 250 preferably provided in the form of a substantially endless rotating drum 100.

The clustering means 250 includes a pattern of perforations 304, preferably in the form inlet regions 304a joined to outlet regions 304b, and are arranged to form and provide a desired printing pattern 320 of particulate material clusters 703 onto the carrier layer 401. The support means 600 is preferably in substantial contact with the support surface 412 of the carrier layer 401. The support means 600 preferably ensures a close enough connection in between the deposit surface 411 of the carrier layer 401 and the outlets regions 304b to prevent unwanted migration of the particulate materials 201 from the carrier deposit zones 415 to the carrier inter-deposit zones 416. Although not required to work the current invention, it is highly recommended to foresee a close pressure contact connection in between the deposit surface 411 of the carrier layer 401 and the outlets regions 304c and inter-outlet zones 309 of the clustering means 250 to prevent unwanted migration and/or shifting of the particulate materials 201 from the carrier deposit zones 415 towards the carrier inter-deposit zones 416. The close deposit contact ensures favourable deposit and printing of particulate material 201 onto the deposit surface 411 of the carrier layer 401 without substantially loss of material.

The resultant printing pattern 320 forms desired and substantially spaced-apart particulate material clusters 703, preferable without any substantial amounts of particulate materials 201 in between the particulate material clusters 703. A covering means 500 provides an auxiliary layer 501, such as for instance a liquid-permeable fibrous web such as a non-woven, paper, tissue, woven, fabric, web, perforated film or foil and the like to sandwich said particulate material clusters 703, preferably comprising large amounts of absorbent polymer material, between the carrier layer 401 and auxiliary layer 501. Alternatively the auxiliary layer 501 can also represent a homogenous and/or heterogeneous layer of glue, adhesive, binders, resins, thermoplastic material and the like, capable of sandwiching the particulate materials clusters 703 between the carrier layer and auxiliary layer. This relative expensive, technically challenging and environmentally burdensome alternative embodiment according to the invention is however not preferred above the typical non-woven, paper or tissue like layer for instance.

The clustering means 250 is suitably constructed and arranged to direct a selected flow of particulate material 201 onto the carrier deposit zones 415 of the carrier layer 401. It should be appreciated that other particulate material 201 may also be introduced via the clustering means 250 as desired. A particulate supplying means 200, such as for instance a K-Tron weight and loss feeder, Model N° K10s, type of particulate delivery system can be configured to deliver required amounts of particulate material 201 through a feeding tube 205 into the clustering means 250. The particulate material 201 reach the perforations 304 by means of gravity and/or other physical forces. In particular embodiments, a conventional air-conveying system may be employed to move and guide the particulate material 201 and resultant particulate material stream to the desired locations in the clustering means 250. Alternatively such forces may e.g. be electro-mechanical forces in case the particulate material 201 is responsive to a magnetic field and magnets are placed to guide the particles towards the perforations 304. Alternatively, the particulate material 201 can, for instance, be gravimetric or volumetric assessed, guided and fed indirectly via the feeding tube 205 and/or directly into the clustering means 250 under the influence of gravity without the use of additional conveying air.

Optionally, one can also provide a vacuum suction system in function of the deposit zones 415 of the carrier layer 401, creating an air flow towards the perforations 304 and/or deposit zones 415. Such vacuum suction system would generate suitable levels of vacuum within an appointed vacuum section, and would provide a desired level of vacuum within the clustering means 250. It should be readily appreciated that such particular levels of vacuum generated within clustering means 250 and/or drum 100 would depend upon the individual circumstances of the manufacturing line. For example, at higher rates of rotation of the drum 100, relatively higher levels of vacuum may be required within clustering means 250. In addition, the use of conveying means to transport the particulate material 201 into the clustering means 250 may necessitate the use of even higher levels of vacuum. It should also be readily appreciated that the levels of vacuum shall also depend upon the porosity of the carrier layer 401, and the usage of possible carrier layer 401 would for instance be limited to fibrous web materials and/or perforated films and foils. For the reason above and in light high energy consumption this vacuum suction usage is an alternative embodiment according to the invention, however not one of the most preferred one.

More economically and environmentally beneficial production processes with lower energy usage and more freedom on the choice of carrier layer 401 are preferred. Further to extensive testing it was demonstrated that using gravity will be sufficient for a wide range of particulate material 201 usages at various production speeds.

The particulate material 201 supplied via the particulate material supplying means 200 is preferably provided in uniform particulate material stream, reaching a width equal to or slightly greater than the width 310 of the perforation pattern 300 on the side of the inlet regions 304a. In order to improve uniformity, a vibrating supply means 200 might be used. In a preferred embodiment, a feeding tube 205, with a proximal opening 206 and distal opening 207 can be used to concentrate the particle material stream onto a certain area. Such feeding tube 205 may have various suitable longitudinal and transversal shapes and is preferably designed in light of the dimension and shape of the clustering means 250, its perforation pattern 300, its perforations 304 and the desired printing pattern 320. The feeding tube 205 may also converge towards the clustering means 250 to effectively and efficiently guide the particulate material stream to the inlet regions 304a. The particle supply means 200 preferably contains a continuous supply and a dosing system 204 of a gravimetric, volumetric or other type so as to control the quantity and quality of particles fed into the perforations 304. Such particle supply means are being offered and supplied by Acrison, Inc., having offices in New Jersey, New York.

The particle material 201 such as absorbent polymer material, more preferably absorbent particulate polymer material may be provided and used in various shapes or forms, such as granular, spherical, flakes, fibrous, and will often consist of irregularly shaped particles, having a mean particle size from about 10µm to 1000 µm, preferably with less than about 5% by weight having a particle size of 5 µm, and preferably with less than about 5% by weight having a particle size of more than about 1200 µm. For use in absorbent structures to be used in absorbent articles, an absorbent material will be selected which can swell upon contact with liquids, such as bodily exudates. Such materials can be supplied in a granular form by Evonik from Essen, Germany, BASF from Antwerp, Belgium, Nippon Shokubai from Osaka, Japan and San-Dia from Tokyo, Japan. These are cross-linked polymeric materials that can absorb at least about 5 times their weight of an aqueous 0.9% saline solution as measured using the Centrifuge Retention Capacity test (Edana 441.2-01).

The web supplying means 400 can include a conventional spindle and supply roll controlling mechanism of the type that is well know in the art. For example, suitable spindle and control mechanisms are available from Martin Automatic Inc., a company having offices in Rockford, Illinois.

The carrier layer 401 may preferably be brought in their desired position via guiding systems 402 and the auxiliary layer 501 may preferably be brought in their desired position via the guiding systems 502, and the support means 600 is preferably controllably strained via tensioning means 603 and operated via guiding means 601 and 602. The composite material 700 is preferably guided via guiding means 603 and the desired material 702 is preferably guided by guiding means 604.

The carrier layer 401 can be any suitable material web which has sufficient strength to process through the apparatus, and preferably economically, environmentally and usage sensible. The carrier layer 401 may comprise a paper or fibrous tissue, woven or non-woven fabric, a cellulose web or batt, airlaid or wet laid structure or the like. Alternatively, the carrier layer 401 is a porous, gas permeable web material such as a porous film or fibrous web.

The auxiliary layer 501 can be any suitable material web which has sufficient strength to process through the apparatus, and preferably economically, environmentally and usage sensible. The carrier layer 401 may comprise a paper or fibrous tissue, woven or non-woven fabric, a cellulose web or batt, airlaid or wet laid structure or the like. Alternatively, the carrier layer 401 is a porous, gas permeable web material such as a porous film or fibrous web. Furthermore the auxiliary layer 501 can also represent for instance a spray, film and/or layer of glue, adhesive, binders, resins, thermoplastic material and the like together with the carrier layer 401 capable of sandwiching the particulate material clusters 703.

The carrier layer 401 and/or auxiliary layer 501 may also be an essentially endless web material in the longitudinal direction. One preferred web material is a so called SMS material, comprising a spunbonded, a melt-blown and a further spunbonded layer. Highly preferred are permanently hydrophilic non-wovens, and in particular nonwovens with durably hydrophilic coatings. An alternative preferred material comprises a SMMS-structure. Another preferred web material is a nonwoven containing cellulosic fibers, paper or tissue sheet or other airlaid, drylaid or wetlaid material, as these products greatly improve the wicking capacity of the product. The web materials 401 and 501 may be provided from two or more separate sheets of materials or they may be alternatively provided from a unitary sheet of material. Preferred non-woven materials are provided from synthetic fibers, such as PE, PET and most preferably PP. As the polymers used for non-woven production are inherently hydrophobic, they are preferably coated with hydrophilic coatings.

The clustering means 250 can preferably be mounted onto a or unitary with a drum 100 having perforations 304 arranged in a perforation pattern 300 along its circumferential- and/or widthwise direction and thus directly or indirectly piercing or perforating the drum 100 in the radial direction, from its inner surface 101 to its outer surface 102. Alternatively, according to another embodiment of the invention, the clustering means 250 are replaceably mounted and/or unitary incorporated on a substantially endless belt carried on a system of transporting rollers. Suitable forming belt systems are available from Paper Converting Machine Company, a business having offices located in Green Bay, Wisconsin. Driving means, such as conventional electric motors or the like, are constructed and arranged to rotate or otherwise move and translate the clustering means 250 at a predetermined surface speed along a desired manufacturing direction and speed. The various configurations of the invention can advantageously provide the desired composite structures while operating at high surface speeds of at least about 0.5 m/sec, preferably at least about 1 m/sec, more preferably at least about 3m/sec and most preferably more than 7m/sec.

The carrier layer 401 is delivered onto or adjacent the outer peripheral surface of the clustering means 250 mounted onto or unitary with drum 100. As the drum 100 rotates, the moving surface of the drum 100 transports the clustering means 250 and the support means 600 guides the carrier material 401 in close proximity and past the clustering means 250 in light of the intended deposit of particulate material clusters onto the deposit surface 415 of the carrier layer 401. For the sake of simplicity, the wording 'drum' is used, denominating an essentially round circumference, but it is clear to the person skilled in the art that any type of endless body, provided with the desired perforation pattern 300 can be used. The surface of such body can be rigid such as in perforated aluminium plates, or soft such as in a belt. Preferably, a drum 100 made out of steel frame with reinforced aluminium plates will be used.

With reference to Figure 2, in a preferred embodiment according to the invention the outer surface 102 of the drum 100 can be operationally divided into a series of predetermined article segments 275. Each article segment 275 mounted and incorporated into the drum 100 generally corresponds to an absorbent structure for placement in a single absorbent article. The outer surface 102 of the drum can further comprise a plurality of plate means 260 which comprise the clustering means 250 and are serially positioned and longitudinally spaced along the circumference of the drum 100 so as to provide a series of mountable clustering means 250 able to deposit the particulate material 201 on the carrier layer 401. Preferably the article segment 275, the plate means 260 and clustering means 250 are designed so as to complement the rounded drum 100. The different sections making up the drum 100 are constructed of a material, such as metal, that is sufficiently strong to withstand the forces and stresses encountered during operation.

The perforations 304 can have a variety of shapes including, but not limited to, circular, oval, square, rectangular, triangular, and the like. The perforation pattern 300 shown in Figure 2 is a square grid with regular spacing and size of circular perforations 304. Other perforation patterns 300 can be hexagonal, rhombic, orthorhombic, parallelogram, triangular, rectangular, and any and all combinations and derivations thereof. Alternatively, using an irregular perforation pattern 300 with varying sizes and shapes of the perforations 304, very specific and/or complex resultant printing patterns 320 can be made. The spacing between the grid lines 303 may be regular or irregular. Alternatively the configurations of the perforation 304 can also be arranged with one or more elongate shapes positioned with their relatively longer axes aligned at selected angles which diverge or converge to the centerline of the perforation pattern 300. The desired perforation 304, and their respective inlet region 304a and outlet region 304b volumes, are furthermore formed by height 305 of the individual clustering means 250.

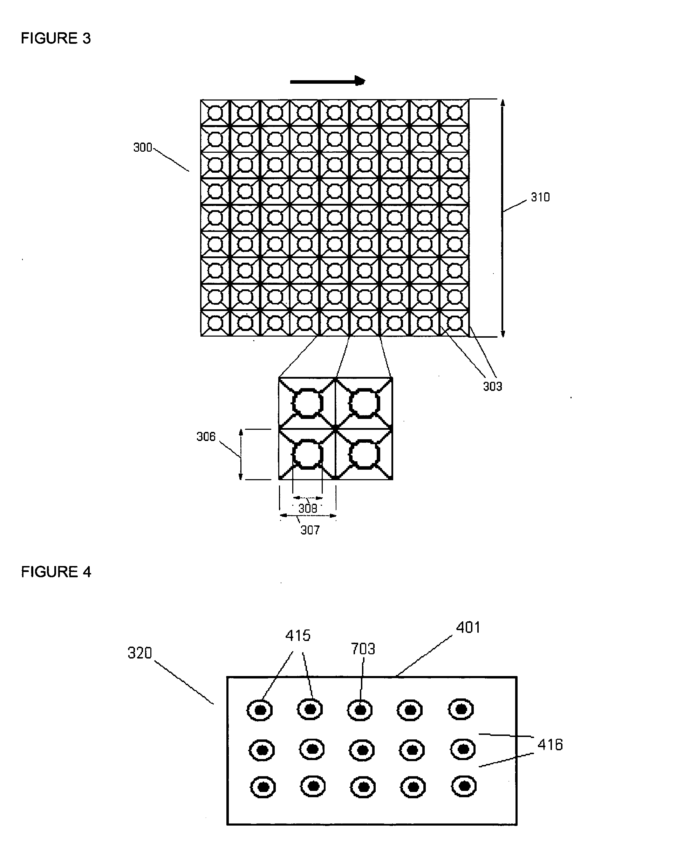

With reference to Figure 4, the perforation patterns 300 will determine the printing patterns 320, whereby the particulate materials 201 will be brought to the carrier deposit zones 415 on the carrier layer 401, preferably not onto the carrier inter-deposit zones 416.

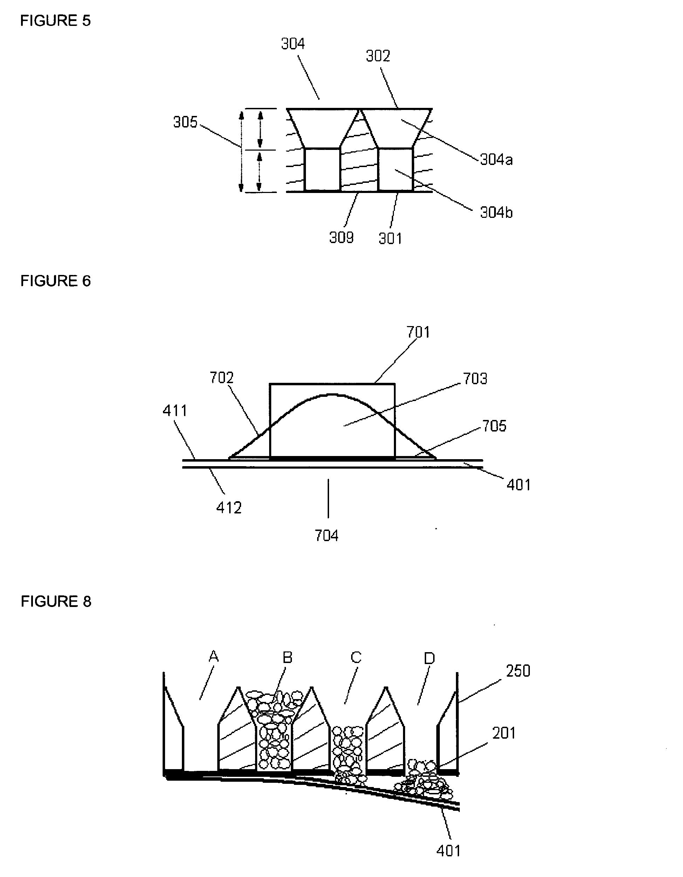

With reference to Figure 5, clustering means 250 includes a pattern of perforations 304 separated from one another by inter-perforation zones, being preferably inter-inlet ridges 303 on the feeding side of the clustering means 250 and inter-outlet zones 309 on the deposit side of the clustering means 250. The perforations 304 have a top opening 302, corresponding to the inner surface 101 of the drum, a bottom opening 301, corresponding to the outer surface 102 of the drum, and a height 305. Top opening 302 and bottom opening 301 can vary in size and shape from each other, where the top opening 302 is in this instance rectangular with transversal side 306 and longitudinal side 307 and the bottom opening 301 is in this instance circular with diameter 308. In a preferred embodiment, the top opening 302 is larger than the bottom opening 301, thus creating a converging funnel structure in the inlet region 304a, allowing the efficient collection of particle material 201 which slides easily into the outlet region 304b. In a preferred embodiment, the top opening 302 are designed as such that the inter-inlet ridges 303 between adjacent top openings 302 are very sharp and narrow so that substantially no particulate material 201 can remain and/or pile up on these inter-inlet ridges 303 and thus substantially all particulate material 201 is collected by the perforation 304.

If the perforations 304 are overfilled, i.e. if more particulate material 201 is present than the perforations 304 can take or than can instantaneously be evacuated via the perforations 304, one has 'overfill'. Particulate material 201 will then accumulate and rise until above the level of the top opening 302 of the perforation 304. In that case, a sweeping means 208 will level the surface of the particulate material 201 with that of the inner surface 101 of the drum 100 and thus remove the excess particulate material 201 so that only a volume of particulate material 201 essentially corresponding to the volume of the perforation 304 is being transferred to the carrier material 401 during depositing. The sweeping means 208 can be for instance a scraper bar made out of durable material, or, as depicted in Figure 1, a rotating brush or wheel 208 mounted closely above the inner surface 101 of the drum 100, or any other suitable solution fit for the purpose. Such sweeping 208 means will also evacuate any particulate material 201 present on the ridges 303 between the adjacent top openings 302. If the perforations 304 are under-filled, i.e. in case the particulate material supply means 200 feeds less particulate material 201 (by volume) into the perforations 304 of the clustering means 250 than can be evacuated during the depositing process, one has 'under-fill'. This is preferred in application, especially where it is important to have the correct weight of particulate material 201, rather than working via the volume of the particulate material 201.

As explained above, the overfilling method functions with sweeping means can act as a volumetric dosing system, where the prescribed volumes, dimensions and shapes of the perforations 304 act as the volumetric dosing and printing space and gives excellent results if the particulate material 201 is very homogeneous in density and particle size, or if the weight of the dosed particles is less relevant than their volume.

However, in many cases it is required to dose the correct weight, rather than the corresponding volume. Such particulate material 201 often have irregular densities by nature and/or such irregular densities are often aggravated by handling and transportation whose vibrations cause the particulate material 201 to segregate, bringing the lower density particles on top and the higher density particles below. Using a volumetric method could then result in important weight differences from one product to another which is undesirable, making gravimetric dosing preferably.

Carrier layer 401 which is delivered by the web supplying means 400 onto the support means 600 delivers the carrier layer 401 in close proximity of and preferably against the outer periphery of the clustering means 250. If the clustering means 250 and the carrier material 401 are pressed to one another they tightly seal off the outlet regions 304b and when preferably moving at essentially zero speed difference to one another, the particulate materials 201 fed by the supply means 200, preferably guided via feeding tube 250, are able to be continuously and controllably deposited onto the clustering means 250 into the perforation 304 via the intlet regions 304a thereby creating the particulate material printing pattern 320 on the deposit zones 415 of the carrier layer 401. Optionally, the particulate material clusters 703 are immobilised, bond, joined and/or otherwise secured restrained in relation to the carrier layer via any suitable means, such as for instance glue, binder, sprays, films, network, webs, etc.

Due to the gravity or other forces, the particulate material 201 will exit the perforations 304 via their bottom opening 301, thereby providing the desired printing pattern 320. This desired printing pattern 320 is substantially retained when the drum 100 separates from the carrier material 401. In the absence of a relative close seal in between the outer surface of the clustering means 250 and the deposit surface 411 of the carrier material 401, a gap 103 will create a possible blurring effect of the desired printing pattern 320, since the drum 100 and carrier layer 401 move at significant speeds and allow migration of the particulate materials 201. Therefore it is recommended to keep the gap 103 a small as technically acceptable.

However, even with a very small gap 103, it is obvious to the person skilled in the art that, depending on the nature of the particulate material 201 and on the amount of particulate material 201 one wants to deposit via one single perforation 304, the actual resulting printing pattern 320 might differ slightly from the perforations pattern 300. The effect is caused by gravity which might pull the particulate material clusters 703 apart so that the particulate material footprint 705 on the carrier layer 401 is wider and/or larger than the bottom opening 301 of the perforations 304 and the forces of inertia might further distort the printing pattern 320 in the direction of the web movement, thereby creating a skewed image 704.

Figure 6 illustrated how, due to the various forces mentioned above, for a set of printed particulate material clusters 703, the printed shape 702 might differ from the original shape 701 which the particulate materials 201 had while still caught in the perforation 304 of the clustering means 250 before being deposited on the carrier layer 401. A careful design of sizes, locations and shapes of the perforations 304 and related dimensions, shapes and rotating speed of the clustering means 250 and drum 100 will ensure satisfying results after having evacuated the particulate material 201 onto the carrier material 401.

The composite structure 700 as a combination of the auxiliary layer 401 and the particulate material clusters 703 is moved away from said clustering means 250 via a transport means, while in case required a stabilising means ensures the particulate material clusters 703 are not unacceptably distorted, the particulate materials 201 do not unintentionally migrate to the inter-deposit zones 416 and/or are lost or wasted due for instance high machine speeds, air and material flows, gravitational and other forces and the like. In a preferred embodiment the composite structure 700 is immediately stabilised when taken over from the carrier material support means 600 by for instance opposing contact pressured longitudinal conveyer belts, vacuum suction means, or the like. Transport means can be unitary with stabilisation means.

As a next step, one does immediately bond or join a second web material such as for instance an auxiliary layer 501 directly or indirectly unto the carrier layer 401 and the particulate material clusters 703 deposited thereon according to printing pattern 320, and the resulting sandwich structure is additionally preferably secured via an ultrasonic attachment means 800 so that the particulate materials 201 are sandwiched, immobilized, bonded, joined and/or restrained in between the carrier layer 401 and auxiliary layer 501 in their desired location and shapes by means of the thereby formed pockets or compartments.

Alternatively the auxiliary layer 501 can for instance also comprise a liquid-permeable layer or web material, such as films, foils, tissues, fabrics, webs or the like. The layer may for instance comprise paper, tissue, wet laids or drylaids, wovens or non-woven and/or may be composed of for instance hydrophilic materials or composed of a hydrophobic material which has been suitably treated to render it sufficiently hydrophilic..

Additionally the composite structure 700 and/or absorbent structure can be alternatively and/or complementary be further combined with any and all other suitable covering, absorbency feature enhancing, protective, functional, structural and/or strengthening materials, fabrics and/or webs (e.g. acquisition layer, surge layer, distribution layer, wicking layer, backing layer, elastic layer, colored layer, perfumed layer, lotion layer, informative layer, etc) and the like.

Auxiliary layer 501 can preferably be bound or joined to the carrier layer 401 with the particulate material clusters 703 immobilized and/or restrained there between via attachment means 800 such as for instance ultra sonic and/or other thermal, mechanical or thermomechanical bonding techniques. Such discrete bonds or joints in between the carrier layer 401, particulate material clusters 703 and/or auxiliary layer 501 can preferably be provided in one or more of the inter-deposit zones 416 in the form of grids, patterns, lines, dots and the like.

In a particular aspect of the invention, the ultrasonic attachment means 800 can be configured to creating substantially permanent primary attachments and substantially detachable secondary attachments leading to a substantially water-sensitive absorbent structure. The resultant water-sensitive attachment means provides an attachment system wherein the strength of the attachment means is great enough to adequately hold the carrier layer 401 and the auxiliary layer 501 together when the system is substantially dry and also when the absorbent structure is partially or fully wetted. In addition, the wet-strength of the attachment system is configured to be sufficiently low so as to not excessively constrict the swelling expansion of absorbent particulate polymer materials during the absorption of liquid. The wet-strength of the attachment means is preferably less than the separating force imparted by the swelling of the high absorbency material when the absorbent particulate polymer material is exposed to liquids. In addition, the water-sensitive attachment system is preferably configured to release at an applied load which is less than the load needed to delaminate the water-sensitive attachment means without excessively tearing the material forming either or both of the carrier layers 401 and/or auxiliary layer 501 when such layers are wetted. The water-sensitive attachment system is preferably also configured to release at an applied load which is less than the load needed to excessively burst the material forming either or both of the outer layers of the absorbent structure when wetted. Typically, the applied load is a generally tensile load resulting from the pressure exerted by the expanding absorbent particulate polymer material when the particulate material absorbs liquid and swells. The appropriate attachment means are constructed and arranged to be sufficiently strong to withstand this pressure and substantially avoid bursting or tearing. Such ultrasonic bonding system can for instance be supplied by Herrmann, from Germany.

The attachment means for securing together carrier layer 401 and auxiliary layer 501 may also be constructed to provide any other suitable connecting mechanism, such as thermal bonds, sonic bonds, stapling, stitching, or the like. The attachment means 800 can preferably be configured to provide operable pockets particulate material clusters 703 between the carrier layer 401 and auxiliary layer 501. Optionally, a perimeter means can be included in the process to obtain a mechanism for providing substantially continuous side attachment regions, and a selected pattern of intermittent, longitudinally-spaced, medial attachment regions. The perimeter attaching means may be configured to provide other types of securing, such as sonic bonds, thermal bonds, stitching, sewing, or the like.

The auxiliary layer 501 can preferably be provided by covering means 500 and a guiding system 502 of transporting rollers and conveyers which are configured to deliver the auxiliary layer into a contacting relation with the composite structure 700 comprising a carrier layer 401 and the printing pattern 320 of particulate material clusters 703.

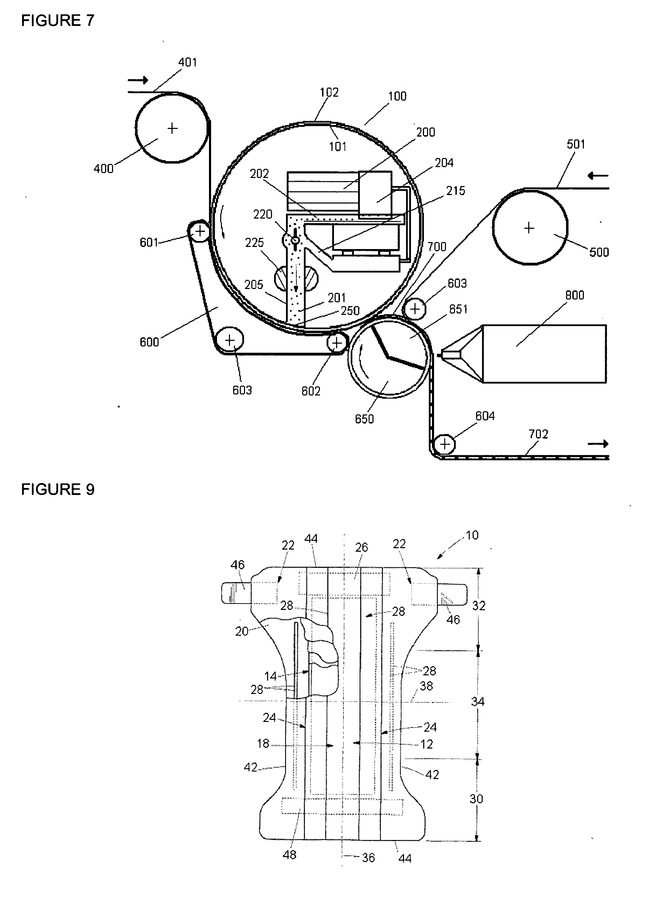

With reference to Figure 7, a preferred embodiment of an apparatus and method according to this invention is illustrated, particularly suitable for manufacturing at very high speed super absorbent particle sheets as absorbent structures for absorbent articles such as a baby diaper. It includes a particle supply means 200 with feeding tube 205, recovery tube 215 and deflector 220, a clustering means 250 in the form of an essentially endless rotating drum 100, comprising perforations 304 in a desired perforation pattern 300. Below and in close proximity to the rotating drum 100, a moving carrier layer 401 is arranged onto an essentially endless support means 600, preferably moving at essentially the same speed as the rotating drum 100. Particles 201 falling out of the feeder means 200 in the drum 100 and onto the clustering means 250 into the perforations 304, thereby catching and gathering the desired amount of particulate material 201 for building up the particulate material clusters 703 which are to be deposited according to printing pattern 320. Via a takeover drum 650, with suction segment 651, the composite structure 700 of carrier layer 401 with printed pattern 320 is removed form the clustering means 250 and drum 100 before being covered by an auxiliary layer 501. A stripper roll can also be employed to help separate the composite structure 700 from the drum 100. An ultrasonic attachment system 800 is provided to bond and/or join the various layers of material to one another and preferably creating the predefined pockets and compartments with the particulate material clusters 703 by way of providing permanent and/or detachable bonds and/or joint within the inter-deposit zones 416, preferably throughout the absorbent polymer particulate material area. The suction segment 651 retains the particulate material 201 into position until it has been sandwiched and fixed by the ultrasonic sealing, creating the desired material 702. The takeover drum 650 preferably functions also as anvil for the ultrasonic tool. After the assembling operation has formed the desired material 702, the absorbent structure is in a preferred embodiment of the invention directed to other areas of the apparatus for further processing.
Creating constantly and quickly repeating pulses of particulate material 201 suspended in a conveyer means 225 such as air has been a long lasting desire for many applications, in particular for pulses which are well controlled both with regard to their shape, their frequency and the amount of material transferred during these pulses. A particular useful application is during the manufacture of disposable absorbent articles, such as feminine hygiene garments, baby diapers, baby pants and/or adult incontinence garments and the like, where the manufacturing aims at high production speed and low variability. This can be achieved by means of the deflector 220 which will interrupt the particle material stream by pushing the particulate material 201 into a recovery tube 215 and can thus create pulses of particulate material 201. The advantage is that one can thus choose not to print certain segments of the drum 100, i.e. leave certain areas of the perforation pattern 300 void of particle material. Also, the unprinted segments of the drum 100 can vary from one rotation of the drum to another, thereby saving enormously on the investment cost of providing different drums 100 with different perforation pattern 300, and on the maintenance and production cost of changing such different drums 100. As an example: with this solution, it is possible to manufacture composite structure 700 or absorbent for the use in absorbent articles such as diapers in all sizes, from the smallest one to fit a newborn baby unto the bigger ones for toddlers, on just one drum 100, which is not possible with conventional techniques known in the art and is highly appreciated and advantages.

Figure 8 provides schematic enlarged sectional view of a part of the clustering means 250 depositing particulate material 201 via the perforations 304 onto their deposit region 415 according to a printing pattern 320 according to the process shown in Figure 7. The perforations 304 are shown in four exemplary and non-limitative different states during the method according to the present invention; the first state "A" being the empty phase, just before loading of the perforations 304 or after having fully evacuated particulate material 201 there from; the second state "B" being the fully loaded state whereby the perforations 304 have collected particulate materials 201 so as to fill the perforation 304; the third state "C" being the start of the depositing process (partially) whereby the carrier layer 401 and the outer surface of the clustering means 250 are gradually moving away from each other such as is for instance the case when the substantially endless rotating drum 100 is departing from the carrier layer 401 thereby allowing partial evacuation of the particulate material 201; the last state "D" being the essentially full deposit of the particulate material cluster 703 onto the carrier layer 401 so as to, after full evacuation of the particulate material 201 onto the carrier layer 401, to return back to state "A", again allowing catching and collection of particulate materials 201 filling the void space for another particulate material cluster 703 depositing and printing pattern 320 process.

Figure 9 is a top plan view of a diaper 10 as a preferred embodiment of an absorbent article including an absorbent structure according to the present invention. It should be understood, however, that the present invention is also applicable to other absorbent articles such as feminine hygiene garments, baby pants, adult incontinent garments and the like.

The absorbent article is shown in its flat out, un-contracted state with the wearer side facing the viewer. Portions of the absorbent article are cut away to more clearly show the underlying structure of the diaper 10 including the absorbent elements and absorbent components. The chassis 12 of the diaper 10 in Figure 9 comprises the main body of the diaper 10. The chassis 12 comprises an outer covering including a liquid pervious top sheet 18 and/or a liquid impervious back sheet 20. The chassis 12 may include a portion of an absorbent structure 14 encased between the top sheet 18 and the back sheet 20. The chassis 12 may also include most or all of the absorbent structure 14 encased between the top sheet 18 and the back sheet 20. The chassis 12 preferably further includes side panels or ears 22, elasticized leg cuffs 24 and elastic waist features 26, the leg cuffs 24 and the elastic waist feature 26 each typically comprise elastic members 28. One end portion of the diaper 10 is configured as a front waist region 30 of the diaper 10. The opposite end portion is configured as a back waist region 32 of the diaper 10. An intermediate portion of the diaper 10 is configured as a crotch region 34, which extends longitudinally between the first and second waist regions 30 and 32. The waist regions 30 and 32 may include elastic elements such that they gather about the waist of the wearer to provide improved fit and containment (e.g. elastic waist feature 26). The crotch region 34 is that portion of the diaper 10 which, when the diaper 10 is worn, is generally positioned between the wearer's legs. The diaper 10 is depicted with its longitudinal axis 36 and its transverse axis 38. The periphery of the diaper 10 is defined by the outer edges of the diaper 10 in which the longitudinal edges 42 run generally parallel to the longitudinal axis 36 of the diaper 10 and the end edges 44 run between the longitudinal edges 42 generally parallel to the transverse axis 38 of the diaper. The chassis 12 also comprises a fastening system, which may include at least one fastening or securing member 46 and at least one landing zone 48. The various components within the diaper 10 may be bound, joined or secured by any method know in the art, for example by adhesives in uniform continuous layers, patterned layers or arrays of separate lines, spirals or spots. The top sheet 18, the back sheet 20, the absorbent structure 14 and other components may be assembled in a variety of well-known configurations and are well known in the art.

The back sheet 20 covers the absorbent structure 14 and preferably extends beyond the absorbent structure 14 toward the longitudinal edges 42 and end edges 44 of the diaper 10 and may be joined with the top sheet 18. The back sheet 20 prevents the bodily exudates absorbed by the absorbent structure 14 and contained within the diaper 10 from soiling other external articles that may contact the wearer, such as bed sheets and undergarments. In preferred embodiments, the back sheet 20 is substantially impervious to bodily exudates and comprises a laminate of a non-woven and a thin plastic film such as a thermoplastic film. The back sheet 20 may comprise breathable materials that permit vapour to escape from the diaper 10 while still preventing bodily exudates from passing through the back sheet 20. It may be semi-rigid, non-elastic and can be made fully or partially elasticized and include backing. The back sheets 20 may be assembled in a variety of well-known configurations and are well known in the art.

The diaper 10 comprises a top sheet 18 that is preferably soft, compliant, exhibits good strikethroughs and has a reduced tendency to rewet from the liquid absorbent material. The top sheet 18 is placed in close proximity to the skin of the wearer when the diaper 10 is worn. In this way, such top sheet 18 permits bodily exudates to rapidly penetrate it so as to flow toward the absorbent structure 14 more quickly, but preferably not allowing such bodily exudates to flow back through the top sheet 18. The top sheet 18 may be constructed from any one of a wide range of liquid and vapour permeable, preferably hydrophilic, materials. The upper and lower surface of the top sheet 18 may be treated differently and may for instance include a surfactant on the upper surface so as to facilitate liquid transfer there through, especially at a central zone or area of the top sheet 18 located over the absorbent structure 10, and for instance include a hydrophobic agent on the lower surface to minimize the liquid contained within the absorbent core from contact wetting the top sheet 18 thereby reducing rewet values. The top sheet 18 may also be coated with a substance having rash preventing or rash reducing properties (e.g. aloe vera). The top sheet 18 covers substantially the entire wearer facing area of the diaper 10, including substantially all of the front waist region 30, back waist region 32, and crotch region 34. Further, the side panels 22 and/or waist feature layers of the inner region may be formed from the same single top sheet material and, thus, may be referred to as being unitary with the top sheet 18 in forming longitudinal and lateral extensions of the top sheet 18 material. Alternatively, the top sheet 18 may be formed from multiple different materials which vary across the width of the top sheet 18. Such a multiple piece design allows for creation of preferred properties and different zones of the top sheet 18. The top sheet 18 be semi-rigid, non-elastic and can be made fully or partially elasticized. The top sheet 18 may be assembled in a variety of well-known configurations and are well known in the art.

The absorbent structure 14 in Figure 9 generally is disposed between the top sheet 18 and the back sheet 20. The absorbent structure 14 may comprise any absorbent material 110 that is generally compressible, conformable, non-irritating to ,the wearer's skin, and capable of absorbing and retaining bodily exudates. The absorbent structure 14 may comprise a wide variety of liquid absorbent materials 110 commonly used in absorbent articles such as fluff pulp, which is generally referred to as airlaid. Examples of other suitable absorbent materials include creped cellulose wadding; melt blown polymers; chemically stiffened, modified or cross-linked cellulosic fibres; tissue, including tissue wraps and tissue laminates; absorbent foams; absorbent sponges; absorbent polymer materials; absorbent gelling materials; or any other known absorbent materials or combinations of materials. The absorbent structure 14 may further comprise minor amounts (typically less than 10 %) of non-liquid absorbent materials, such as adhesives, binders, plastics, waxes, oils and the like. The absorbent structure 14 according to various embodiments of the invention may be configured to extend substantially the full length and/or width of the diaper 10. However, alternatively the absorbent structure 14 according to the invention is not coextensive with the entire diaper 10 and is limited to certain regions of the diaper 10 such as for instance the crotch region 34. In various embodiments, the absorbent structure 14 extends to the edges of the diaper 10 and the absorbent material 110 is concentrated in the crotch region 34 or another target zone of the diaper 10. In still another embodiment, the particles can be a combination of absorbent material 110, preferably comprising absorbent polymer material, and skin care particles such as ion exchange resins, deodorant, anti-microbial agents, binder particles, or other beneficial particles.

The diaper 10 may also utilize a pair of containment walls or cuffs 24. Each cuff 24 is a longitudinally extending wall structure preferably positioned on each side of the absorbent structure 14 and spaced laterally from the longitudinal axis 36. The longitudinal ends of the cuffs 24 may be attached or joined, for example, to the top sheet 18 in the front and rear waist regions 30 and 32. Preferably, the ends of the cuffs 24 are tacked down inwardly and attached, for example, by adhesive or sonic bonding to the lower structure. Such a construction effectively biases the cuffs 24 inwardly and is generally considered to cause the cuffs 24 to exhibit improved leakage prevention properties. Preferably, the cuffs 24 are equipped with elastic members 28, which extend along a substantial length of the cuffs 24. In a common application, the elastic members 28 are placed within the cuffs 24, preferably at the top of the cuff 24 while in a stretched condition and then glued or sonic bonded to the cuff 24 at least at their ends. When released or otherwise allowed relaxing, the elastic members 28 retract inwardly. When the diaper 10 is worn, the elastic members 28 function to contract the cuffs 24 about the buttocks and the thighs of the wearer in a manner, which forms a seals between the diaper 10, the buttocks and the thighs. The cuffs 24 may be assembled in a variety of well-known configurations and are well known in the art.

The diaper 10 may also employ additional layers known in the art including an acquisition layer or surge layer, preferably situated between the top sheet and the absorbent core and highloft and/or coverstock layers. This serves to slow down the flow so that the liquid has adequate time to be absorbed by the absorbent core.

In order to keep the diaper 10 in place about the wearer, preferably at least a portion of the back waist region 32 is attached by fastening or securing members 46 to at least a portion of the front waist region 30, preferably to form leg openings and an absorbent article waist. Fastening or securing members 46 carry the tensile load around the absorbent article waist and compliment the elastic members 28 by providing a quasi-seal between the wearer, the elastic waist feature 26 and cuffs 24, so that bodily exudates are contained within the diaper 10 which are then absorbed. In other words, so that it does not leak through gaps between the wearer and the edge of the diaper 10. The fastening or securing members 46 may for instance be adhesive, mechanical fasteners, hook and loop features, conceivable strings and/or combinations thereof, i.e., anything that will secure one end of the diaper 10 to the longitudinally opposite end of the diaper 10. The fastening or securing members 46 may also be co-adhesive such that they adhere to each other but not other materials. The fastening or securing members 46 and any component thereof may include any material suitable for such a use, including but not limited to plastics, films, foams, non-woven webs, woven webs, paper, laminates, fibre reinforced plastics and the like, or combinations thereof. It may be preferable that the materials making up the fastening or securing members 46 are flexible, extensible and/or elastic, allowing them to better conform to the shape and movements of the body and thus, reduces the likelihood that the fastening system will irritate or injure the wearer's skin. Preferably, the diaper 10 is affixed to the wearer by tape fasteners which are permanently affixed to the back sheet 20. Tape fasteners are contacted with the transversely opposite side panel or ears 22 attached or joined and extending from the back sheet 20, where they remain affixed due to the binding compound applied to the fasteners. Alternatively, the absorbent article may be pants and the like. In this configuration, the absorbent article may or may not have tape fasteners. Specific disposability tapes may however also be provided on such absorbent articles. All fastening and securing elements 46 may be assembled in a variety of well-known configurations and are well known in the art.

The waist regions 30 and 32 each comprise a central region and a pair of side panels or ears 22 which typically comprise the outer lateral portions of the waist regions. These side panels 22 may be unitary with the chassis 12 and/or back sheet 20 or may be attached or joined thereto by any means know in the art. In a preferred embodiment of the present invention, the side panels 22 positioned in the back waist region 32 are flexible, extensible and/or elastic in at least the lateral direction (i.e., elasticized side panels), in another embodiment the side panels 22 are non-elastic, semi-rigid, rigid and/or stiff. This variety of side panels 22 are well known.

Furthermore waistbands 26 employing elastic members can be positioned along the transverse portion of the diaper 10 so that when worn, the waistbands 26 are positioned along the waist of the wearer. Generally, the waistband 26 preferably creates a seal against the waist so that bodily exudates do not leak from the regions between the elastic waistband 26 and the waist of the wearer. Although the bodily exudates are primarily absorbed by the absorbent materials within the diaper 10, the seal is important considering the assault of liquid by the wearer may overwhelm the absorption rate capacity of the absorbent structure 14. Hence, the waistbands 26 contain the liquid while it is being absorbed, they are well known in the art. The absorbent article such as a diaper 10 may also include such other features, components and elements as are known in the art including front and rear ear panels, waist cap features, elastics and the like to provide better fit, containment and aesthetic characteristics. These features may be assembled in a variety of well-known configurations and are well known in the art.

## Claims

1. A method for producing a sandwich structure comprising a pattern of particulate material, said method comprising the steps of:
a - providing at least one essentially endless layer as carrier layer and/or auxiliary layer
b - providing an essentially endless support means for said carrier layer,
c - positioning said carrier layer over said support means,
whereby said carrier layer is in contact with the contact surface of said support means, and whereby the relative speed between said carrier layer, contact surface of
said carrier support means and the clustering means is essentially zero;
d - providing a particulate material stream from a particle material supply means;
e - directing the particulate material through the perforations of a clustering means;
f - providing an essentially endless material as auxiliary material;
g - combining said auxiliary layer and said carrier layer with said particulate material sandwiched there between;
**characterized in that,**
g- said clustering means deposits said particulate material in a pre-designated locations onto the carrier layer thereby creating a printing pattern of particulate material onto said carrier layer, thereby forming a primary pattern of particulate material and
i - providing an ultrasonic or heat sealing sandwich immobilisation means,

2. A method according to claim 1, **characterized in that** the method comprises the step of removing from the clustering means those particulate material which have not been caught by the perforations of the clustering means.

3. A method according to claim 1, **characterized in that** the method comprises evacuating substantially all particulate materials via the bottom openings of the clustering means.

4. A method according to any one of claims 1-3, **characterized in that** the clustering means is a rotating drum.

5. A method according to any one of claims 1-4 **characterized in that** the method comprises interrupting the particle material stream to create areas where no particulate materials have been deposited.

6. A method according to any one of claims 1-3, **characterized in hat** the method comprises placing an auxiliary layer on top of carrier layer on which particulate materials have been deposited so as to form a sandwich structure.

7. A method according to any one of claims 1-4, **characterized in that** the method comprises immobilising and/or restraining the particulate material to the carrier layer and/or auxiliary layer.

8. A method according to any one of claims 1-5, **characterized in that** at least one layer comprises non woven, paper or tissue.

9. An apparatus for producing a sandwich structure comprising particulate material, said apparatus comprising:
a - a clustering means having a desired perforation pattern
b - a particulate material supplying means positioned to provide particulate materials into the inlet regions of the perforations of said clustering means;
c - a carrier layer supplying means for providing a carrier layer; support means for moving said carrier layer in close proximity to the outlet regions of the clustering means;
d - transport means for moving said carrier layer with particulate material clusters away from the clustering means.
e ― covering means for providing an auxiliary layer over said particulate material clusters.

10. An apparatus according to claim 9, **characterized in that** at least one covering means for providing an auxiliary layer over said particulate material clusters is provided.

11. An apparatus according any one of claims 9-10, **characterized in that** at least one ultrasonic bonding means is provided.

12. An apparatus according any one of claims 9-11, **characterized in that** the clustering means is provided in the from of an endless rotating drum.
